# EUROPEAN PATENT SPECIFICATION

(11) **EP 3 156 101 B1**
(45) Date of publication and mention of the grant of the patent: **22.05.2019**
(21) Application number: 15809175.1
(22) Date of filing: 16.06.2015
(51) Int. Cl.: A61N 5/10

(54) **RADIATION-THERAPY SIMULATION APPARATUS**
STRAHLENBEHANDLUNGSSIMULATIONSVORRICHTUNG
APPAREIL DE SIMULATION DE RADIOTHÉRAPIE

(30) Priority: 16.06.2014 JP 2014123243
(43) Date of publication of application: 19.04.2017
(73) Proprietor: Sumitomo Heavy Industries, Ltd., Tokyo 141-6025 (JP)
(72) Inventor: YAMAGUCHI, Takashi, Yokosuka-shi Kanagawa 237-8555 (JP)
(74) Representative: Louis Pöhlau Lohrentz
(86) International application number: PCT/JP2015/067315
(87) International publication number: WO 2015/194554

(56) References cited:
- JP-A- 2012 088 771
- JP-A- 2013 544 605
- US-A1- 2011 153 547

## Description

### Technical Field

The present invention relates to a radiation-therapy simulation apparatus.

### Background Art

When radiation therapy is performed, it is necessary to plan an optimal irradiation condition for reducing an irradiation dose for a normal tissue in the vicinity of a tumor and thus increasing an irradiation dose for the tumor prior to the therapy. Therefore, in recent years, simulation using a Monte Carlo method capable of accurately modeling behaviors of particles has been performed. Regarding computation of a dose distribution using the Monte Carlo method in radiation therapy of the related art, a three-dimensional model of a human body is created on the basis of medical image data such as computed tomography (CT), magnetic resonance imaging (MRI), or positron emission tomography (PET) of a patient, and is converted into a computation model so that a dose distribution is calculated.

### Citation List

### Patent Literature

[PTL 1] Japanese Unexamined Patent Application Publication No. 2012-88771

Further documents dealing with radiation therapy simulation are for example:
US 2011 153 547, wherein data obtained from previous treatments is used to create and modify a new treatment plan;
WO 2014 063 748 wherein the data stored in organ geometry database is warped to fit the patient image.

### Summary of Invention

### Technical Problem

Since there are parts (body tissues) whose densities are greatly different from each other in the body, a particle which is incident into the human body shows a complex behavior. In the simulation using the Monte Carlo method, an accurate dose distribution can be computed since a behavior of each particle is computed, but a computation time increases. Thus, if an irradiation condition for providing a suitable dose distribution is to be obtained by performing simulation several times after changing irradiation conditions, a total computation time required therefor also necessarily increases.

Therefore, it is necessary to determine an appropriate irradiation condition while preventing an increase in a total computation time. An object of the present invention is to provide a radiation-therapy simulation apparatus capable of preventing an increase in a computation time.

### Solution to Problem

According to an aspect of the present invention, there is provided a radiation-therapy simulation apparatus which calculates a dose distribution based on irradiation applied to a target part of a patient, the apparatus including a storage unit that stores data regarding each of dose distributions obtained when a human body model for a predetermined body type is irradiated with radiation with respect to a plurality of irradiation conditions; an input unit that inputs an irradiation condition applied to the patient when the patient is irradiated with radiation, and body type information of the patient; and a correction unit that corrects data regarding a dose distribution stored in the storage unit on the basis of the body type information of the patient and body type information of the human body model.

In the radiation-therapy simulation apparatus of the present invention, respective dose distributions are computed in advance by irradiating a human body model with radiation with respect to a plurality of irradiation conditions, and the dose distributions computed in advance are stored in the storage unit in correlation with the irradiation conditions. The radiation-therapy simulation apparatus inputs an irradiation condition applied when a patient is irradiated with radiation and body type information of a patient by using the input unit, and corrects data regarding the dose distribution stored in the storage unit on the basis of input body type information of the patient and body type information of the human body model. Consequently, the dose distribution computed in advance can be corrected according to the body type information of the patient, and corrected dose distribution can be used. Thus, since it is not necessary to perform computation using a Monte Carlo method several times in order to find out an irradiation condition providing an optimal dose distribution, it is possible to prevent an increase in a total computation time.

The correction unit may compare CT image information including the body type information of the patient with the body type information of the human body model, transform position information in the body type information of the human body model into position information in the body type information of the patient, and correct the data regarding the dose distribution stored in the storage unit according to the transform of the position information. Consequently, the position information can be transformed so that the body type information of the human body model matches the CT image information including the body type information of the patient, and transform into position information corresponding to the body type information of the patient can be performed by applying the transformed position information to the data regarding the dose distribution stored in the storage unit. As a result, it is possible to calculate data regarding a dose distribution matching a body type of a patient and thus to obtain highly accurate data.

The correction unit may transform position information regarding the irradiation condition applied to the patient into position information in the body type information of the human body model, extract data regarding a dose distribution corresponding to the transformed position information from the data regarding the dose distribution stored in the storage unit, and correct the extracted data regarding the dose distribution by transforming the data into position information based on the body type information of the patient. Consequently, it is possible to transform position information regarding an irradiation condition applied to a patient into position information in body type information of the human body model, extract data regarding the dose distribution obtained when the human body model is irradiated with radiation under the transformed irradiation condition, and perform correction by transforming the dose distribution into position information corresponding to the body type information of the patient. As a result, it is possible to obtain highly accurate data.

### Advantageous Effects of Invention

According to the present invention, the number of times of performing computation can be reduced, and thus it is possible to provide a radiation-therapy simulation apparatus which can prevent an increase in a total computation time.

The invention is defined in the claims, other embodiments being merely exemplary.

### Brief Description of Drawings

FIG. 1 is a block diagram illustrating a radiation-therapy simulation apparatus according to an embodiment of the present invention.
FIG. 2 is a schematic diagram illustrating an exterior of a human body on which radiation therapy is performed.
FIG. 3 is a sectional view illustrating a section which is orthogonal to a longitudinal direction of the human body on which radiation therapy is performed.
FIG. 4A is a schematic diagram illustrating a dose distribution for a human body model stored in a storage unit.
FIG. 4B is a schematic diagram illustrating body type information of a patient.
FIG. 4C is a schematic diagram illustrating a dose distribution after being corrected.
FIG. 5 is a flowchart illustrating process procedures in the radiation-therapy simulation apparatus.
FIG. 6 is an image illustrating a dose distribution in the head of a patient. FIG. 7 is a graph illustrating a relationship between an irradiation dose and a volume.

### Description of Embodiments

Hereinafter, a preferred embodiment of the present invention will be described in detail with reference to the drawings. Similar or corresponding portions are given the same reference numerals throughout the drawings, and repeated description will be omitted.

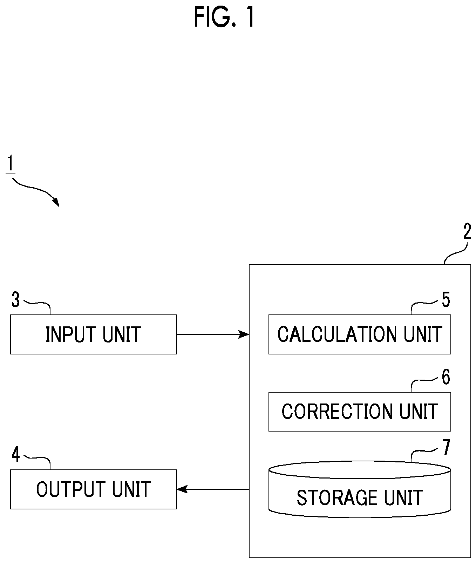

A radiation-therapy simulation apparatus 1 illustrated in FIG. 1 is an apparatus which can be used to create a therapy plan in a radiation therapy apparatus such as a boron neutron capture therapy (BNCT) apparatus. The radiation therapy apparatus includes, for example, an X-ray therapy apparatus, a proton therapy apparatus, and carbon-ion (heavy-ion) therapy apparatus.

The radiation-therapy simulation apparatus 1 calculates a dose distribution of radiation applied to a target part of a patient in radiation therapy. The radiation-therapy simulation apparatus 1 includes a therapy plan terminal 2 for calculating a dose distribution, and the therapy plan terminal 2 is electrically connected to an input unit 3 and an output unit 4.

The input unit 3 inputs various pieces of information required to calculate a dose distribution. The various pieces of information required to calculate a dose distribution include body type information of a patient and information regarding irradiation conditions. The body type information of a patient includes, for example, information regarding positions, sizes and shapes of muscles, bones, organs, tumors, and the like. The input unit 3 may input, for example, computed tomography (CT) image information as the body type information. The input unit 3 may input image information captured by other imaging apparatuses instead of the CT image information. The input unit 3 may be connected to, for example, an imaging apparatus so as to input image information, and may be connected to a recording medium on which image information is recorded, so as to input the image information recorded on the recording medium.

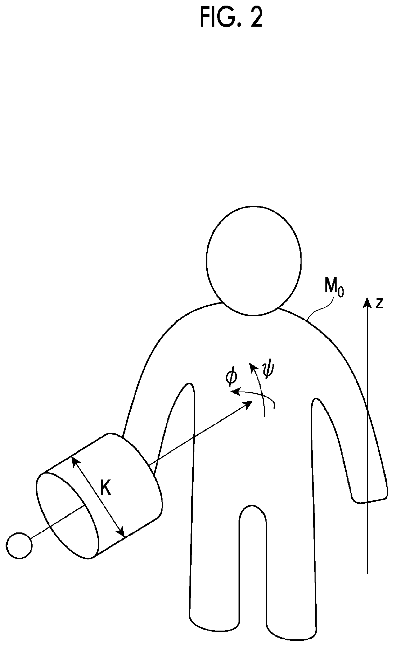

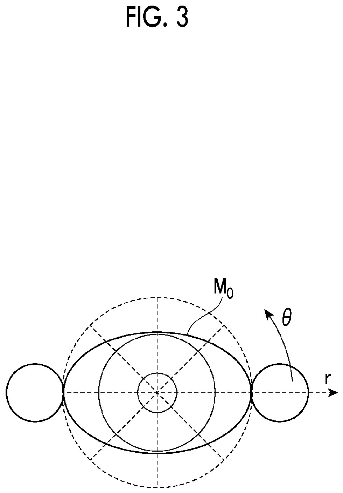

The information regarding irradiation conditions includes, for example, a coordinate (r,θ,z) indicating a position of the isocenter (irradiation target point), an irradiation angle (φ,ψ), the kind of particle to be applied, particle energy, and a collimator diameter K. FIG. 2 is a schematic diagram illustrating an exterior of a human body on which radiation therapy is performed, and FIG. 3 is a sectional view illustrating a section which is orthogonal to a longitudinal direction (body axis direction) of the human body on which radiation therapy is performed. A position of the isocenter is a position at which a particle advancing straight on an irradiation field central axis arrives. For example, a target part such as a treated tumor is disposed at the isocenter. The position coordinate (r,θ,z) of the isocenter may be expressed in a cylindrical coordinate system, and is represented by a radius r, an angle θ, and a height z as illustrated in FIGS. 2 and 3. A process coordinate of the isocenter may be expressed by using other coordinate systems such as an orthogonal coordinate system.

The irradiation angle (φ,ψ) is an angle at which a particle advances toward a target part. For example, the angle φ is an azimuth angle which is an angle about an axis extending in a vertical direction, and the angle ψ is an elevation angle which is an angle about an axis extending in a horizontal direction. The kind of particle to be applied is, for example, a neutron, a proton, or a heavy ion. The energy of a particle to be applied is, for example, kinetic energy of the particle. The collimator is formed of members blocking advancing of a particle, and is used to form an irradiation field by restricting a particle irradiation range. The collimator diameter K is an inner diameter of an aperture through which a particle passes. For example, the collimator diameter K is selected depending on a size of a tumor, and thus a particle irradiation range is restricted.

As the input unit 3, for example, a keyboard, a mouse, a touch panel, a scanner, and other information processing terminals and imaging apparatuses may be used.

The output unit 4 outputs calculation results in the therapy plan terminal 2. As the calculation results, there may be image data indicating a dose distribution. The output unit 4 may include an image display apparatus, a printer, and the like. For example, the calculation results may be output to other information processing terminals.

The therapy plan terminal 2 includes a central processing unit (CPU) performing a calculation process, a read only memory (ROM) and a random access memory (RAM) serving as a storage unit, an input signal circuit, an output signal circuit, a power supply circuit, and the like. The therapy plan terminal 2 includes a calculation unit 5 which calculates a dose distribution, a correction unit 6 which corrects the dose distribution calculated by the calculation unit 5, and a storage unit 7 which stores various pieces of information used to calculate or correct the dose distribution.

In the radiation-therapy simulation apparatus 1, respective dose distributions are calculated in advance by performing irradiation with particles under a plurality of irradiation conditions by using a standard human body model (human body atlas data), and data regarding the calculated dose distributions is stored in the storage unit 7. The storage unit 7 stores body type information of the standard human body model.

The standard human body model is, for example, body type information which is set on the basis of body type information of many people, and includes information regarding shapes and positions of muscles, bones, organs, and the like of persons which are assumed to be standard. Results of calculating a dose distribution obtained by irradiating the human body model with particles in advance are stored in the storage unit 7. The data regarding the dose distribution is stored in correlation with each irradiation condition. The dose distribution for the standard human body model may be calculated by the calculation unit 5 of the radiation-therapy simulation apparatus 1, and may be calculated in advance by other information processing terminals.

Regarding the dose distribution for the standard human body model, respective dose distributions are calculated by irradiating, with particles, a plurality of isocenters which are set, for example, at intervals of 5 cm and 5 degrees from a plurality of directions. The storage unit 7 stores, for example, data regarding dose distributions corresponding to several thousands of irradiation conditions.

The calculation unit 5 calculates a dose distribution by using the Monte Carlo method which is known in the related art. In the Monte Carlo method, a three-dimensional model of a human body is created on the basis of body type information of a human body (for example, CT image information of a patient), and a dose distribution is calculated by converting the three-dimensional model into a computation model.

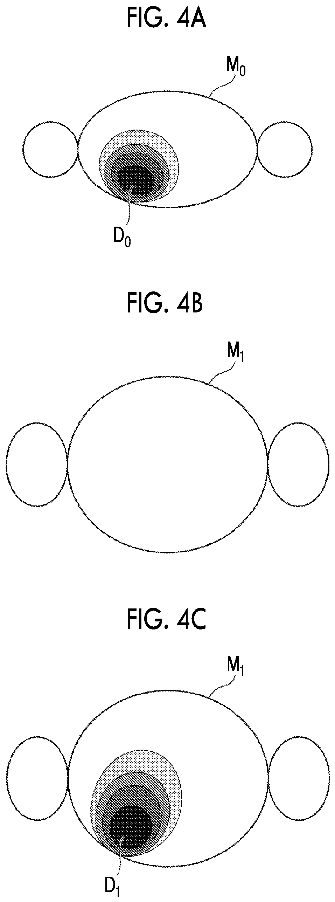

The correction unit 6 corrects data regarding a dose distribution D₀ (standard dose distribution D₀) corresponding to an input irradiation condition on the basis of body type information M₁ (refer to FIG. 4B) of a patient which is input from the input unit 3, and body type information M₀ (refer to FIG. 4A) of the human body atlas data stored in the storage unit 7. The correction unit 6 compares CT image information including the body type information M₁ of the patient with the body type information M₀ of the human body atlas data, transforms position information in the body type information M₀ of the human body atlas data into position information in the body type information M₁ of the patient, and corrects the dose distribution D₀ stored in the storage unit 7 according to the transform of the position information. The correction unit 6 matches the body type information M₀ of the human body atlas data with the body type information M₁ included in a CT image of the patient, and obtains a transform matrix for transforming a position coordinate on the human body atlas data into a position coordinate on the CT image of the patient. The correction unit 6 corrects the data regarding the dose distribution D₀ for the human body atlas data stored in the storage unit 7 by transforming the data into data regarding a dose distribution D₁ corresponding to the body type information M₁ of the patient by using the transform matrix (refer to FIG. 4C).

The correction unit 6 transforms position information in an irradiation condition applied to the patient into position information on the human body atlas data. The correction unit 6 extracts data regarding a dose distribution corresponding to the transformed position information from the data regarding the dose distributions D₀ stored in the storage unit 7. The correction unit 6 corrects the extracted data regarding the dose distribution D₀ by performing transform into position information based on the body type information of the patient.

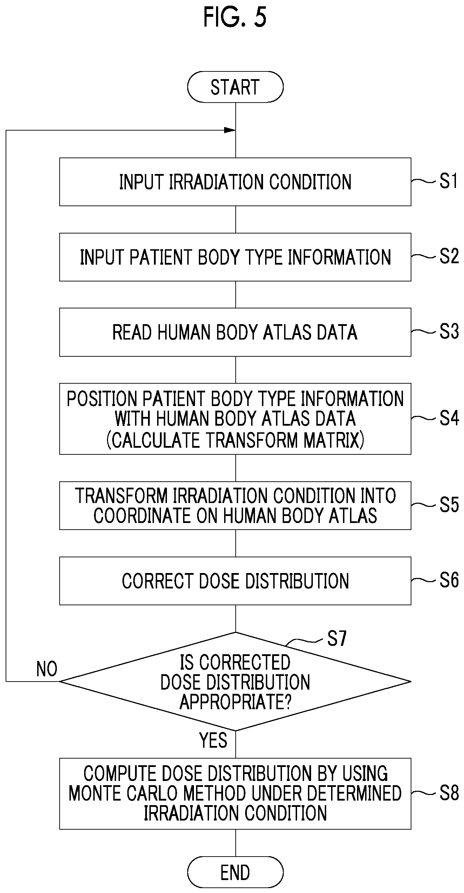

Next, a description will be made of process procedures in the radiation-therapy simulation apparatus 1 with reference to a flowchart illustrated in FIG. 5.

First, a worker inputs irradiation conditions by using the input unit 3 (step S1). Here, the input irradiation conditions are conditions applied when radiation therapy is performed on a patient. Next, the worker inputs body type information of the patient to be treated (step S2) . Here, CT image information of the patient is input from a tomographic image capturing apparatus which functions as the input unit 3.

Next, the correction unit 6 reads body type information of the human body atlas data stored in the storage unit 7 (step S3) . Next, the correction unit 6 performs positioning between the body type information of the patient and the human body atlas data so as to calculate a transform matrix (step S4). At this time, the body type information of the human body atlas data is matched with the body type information of the patient by enlarging or reducing the human body atlas data in any axial line direction.

Next, position information in the irradiation conditions which are input from the input unit 3 is transformed into position information in the human body atlas data (step S5). Here, the coordinate (r,θ,z) indicating a position of the isocenter and the irradiation angle (φ,ψ) as the position information in the irradiation conditions are transformed into position information in the human body atlas data by using the transform matrix.

Next, the correction unit 6 corrects the dose distribution D₀ corresponding to the irradiation conditions (step S6). Specifically, the dose distribution D₀ corresponding to the irradiation conditions transformed in step S5 is extracted, transform into position information corresponding to the body type information of the patient is performed by using the transform matrix with respect to the dose distribution D₀, and thus corrected dose distribution D₁ is calculated.

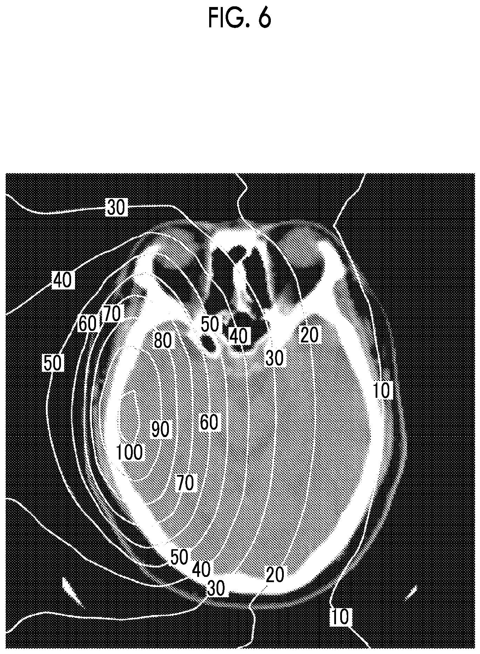

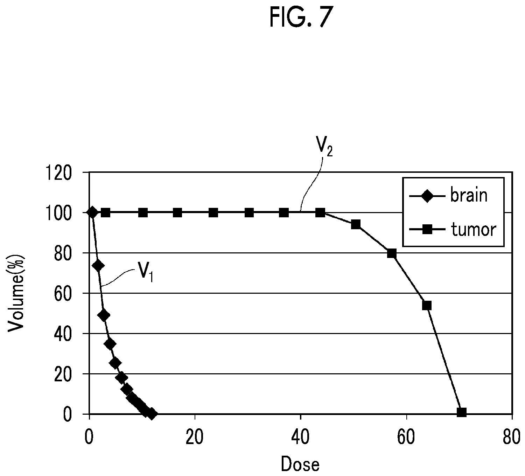

Next, it is determined whether or not the corrected dose distribution D₁ is appropriate (step S7). For example, the calculation unit 5 creates an image of a dose distribution as illustrated in FIG. 6 on the basis of the corrected dose distribution D₁. The calculation unit 5 creates a graph (dose volume histogram) as illustrated in FIG. 7 on the basis of the corrected dose distribution. In the graph illustrated in FIG. 7, a transverse axis expresses a dose, and a longitudinal axis expresses a volume (%). FIG. 7 illustrates a relationship between a dose and a volume assuming that radiation therapy is performed on a tumor in the head of the patient as illustrated in FIG. 6, in which V₁ indicates a volume proportion (%) occupied by normal brain tissues in a radiation irradiation region, and V₂ indicates a volume proportion (%) occupied by a tumor in the radiation irradiation region.

In a line of V₂, the volume is 100% in a range of the dose from about 0 to about 40. In other words, it can be seen that radiation of the dose of about 40 or more is applied to 100% of the tumor portion. In the line of V₂, the volume is about 60% with respect to the dose of about 60. In other words, it can be seen that radiation of the dose of about 60 or more is applied to 60% of the tumor portion. In a line of V₁, the volume is almost 0% with respect to the dose of 10. In other words, it can be seen that radiation of the dose of about 10 or more is not applied to the normal brain tissues. It can be seen from the above description that the tumor portion is irradiated with radiation of a high dose, and the normal brain tissues are irradiated with only radiation of a low dose.

A display unit (liquid crystal display unit) as the output unit 4 displays, for example, an image of the dose distribution illustrated in FIG. 6 or an image of the graph illustrated in FIG. 7. The worker observes the image displayed on the display unit and determines whether or not the corrected dose distribution is appropriate.

In a case where the corrected dose distribution D₁ is not appropriate (step S7: NO), the flow returns to step S1, and the processes in steps S1 to S7 are repeatedly performed. In a case where the corrected dose distribution D₁ is appropriate (step S7: YES), the flow proceeds to step S8. Irradiation conditions corresponding to the corrected dose distribution D₁ are determined as irradiation conditions used for actual treatment.

In the subsequent step S8, the calculation unit 5 computes an accurate dose distribution by using the Monte Carlo method with respect to the determined irradiation conditions. A final result calculated here is output to the output unit 4.

In the radiation-therapy simulation apparatus 1, the respective dose distributions D₀ are computed in advance by irradiating the standard human body model (human body atlas data) with radiation with respect to a plurality of irradiation conditions, and the dose distributions D₀ computed in advance are stored in the storage unit 7 in correlation with the irradiation conditions. The radiation-therapy simulation apparatus 1 inputs an irradiation condition applied when a patient is irradiated with radiation and body type information of a patient by using the input unit 3, and corrects data regarding the dose distribution D₀ stored in the storage unit 7 on the basis of input body type information of the patient and body type information of the human body model. Consequently, the dose distribution D₀ computed in advance can be corrected according to the body type information of the patient, and the corrected dose distribution D₁ can be used. Thus, since it is not necessary to perform computation using the Monte Carlo method several times in order to find out an irradiation condition providing an optimal dose distribution, it is possible to prevent an increase in a total computation time.

The correction unit 6 may compare CT image information including body type information of a patient with body type information of the human body atlas data, transform position information in the body type information of the human body atlas data into position information in the body type information of the patient, and correct data regarding the dose distribution stored in the storage unit 7 according to the transform of the position information. Consequently, the position information can be transformed so that the body type information of the human body atlas data matches the CT image information including the body type information of the patient, and transform into position information corresponding to the body type information of the patient can be performed by applying the transformed position information to the data regarding the dose distribution D₀ stored in the storage unit 7. As a result, it is possible to calculate data regarding a dose distribution matching a body type of a patient and thus to obtain highly accurate data.

The correction unit 6 may transform position information regarding an irradiation condition applied to a patient into position information in body type information of the human body atlas data, extract data regarding the dose distribution D₀ corresponding to the transformed position information from the data regarding the dose distribution D₀ stored in the storage unit 7, and correct the extracted data regarding the dose distribution D₀ by transforming the data into position information based on the body type information of the patient. Consequently, it is possible to transform position information in an irradiation condition applied to the patient into position information in body type information of the human body atlas data, extract data regarding the dose distribution D₀ obtained when the human body atlas data is irradiated with radiation under the transformed irradiation condition, and perform correction by transforming the dose distribution D₀ into position information corresponding to the body type information of the patient. As a result, it is possible to obtain highly accurate data.

It may be checked that an appropriate dose distribution can be obtained under an irradiation condition by correcting data regarding the dose distribution D₀ corresponding to the human body atlas data by using the irradiation condition and body type information of a patient, and then data regarding a dose distribution at the time of applying radiation under the irradiation condition may be obtained by performing computation using the Monte Carlo method. Consequently, it is possible to obtain data regarding a more accurate dose distribution while preventing an increase in a total computation time.

The present invention is not limited to the above-described embodiment, and may be variously modified as follows within the scope without departing from the concept of the present invention.

In the above-described embodiment, a dose distribution in the human body atlas data of a standard body type is calculated in advance, but a human body model may not be a human body model for a non-standard body type. Human body models for a plurality of kinds of body types may be set, and a dose distribution in each human body model may be stored in the storage unit.

In the above-described embodiment, CT image information is used as body type information of a patient, but other examination data may be used as body type information of a patient.

The process procedures illustrated in FIG. 5 may be executed by changing order thereof as appropriate. For example, an irradiation condition may be input after patient body type information is input. An irradiation condition may be input before the irradiation condition is transformed into a coordinate on a human body model.

There may be a configuration in which a terminal including a calculation unit and a terminal including a correction unit are separately provided, and data communication is performed between the terminals.

### Reference Signs List

1 radiation-therapy simulation apparatus, 2 therapy plan terminal, 3 input unit, 4 output unit, 5 calculation unit, 6 correction unit, 7 storage unit

## Claims

1. A radiation-therapy simulation apparatus (1) adapted to calculate a dose distribution based on irradiation applied to a target part of a patient, the apparatus comprising:
a storage unit (7) adapted to store data regarding each of dose distributions obtained when a human body model for a predetermined body type is irradiated with radiation with respect to a plurality of irradiation conditions;
an input unit (3) adapted to input an irradiation condition applied to the patient when the patient is irradiated with radiation, and body type information (M₁) of the patient; and
a correction unit (6) adapted to correct data regarding a dose distribution stored in the storage unit (7) on the basis of the body type information (M₁) of the patient and body type information (M₀) of the human body model,
wherein the correction unit (6) is adapted to compare image information including the body type information (M₁) of the patient with the body type information (M₀) of the human body model, the image information being captured by imaging apparatuses such as CT, to transform position information in the body type information (M₀) of the human body model into position information in the body type information (M₁) of the patient, and to correct the data regarding the dose distribution stored in the storage unit (7) according to the transform of the position information.

2. The radiation-therapy simulation apparatus (1) according to claim 1,
wherein the correction unit (6) is adapted to transform position information regarding the irradiation condition applied to the patient into position information in the body type information (M₀) of the human body model, to extract data regarding a dose distribution corresponding to the transformed position information from the data regarding the dose distribution stored in the storage unit (7), and to correct the extracted data regarding the dose distribution by transforming the data into position information based on the body type information (M₁) of the patient.

## Patentansprüche

1. Strahlungstherapie-Simulationsvorrichtung (1), die ausgelegt ist, eine Dosisverteilung auf Grund einer Strahlung, die auf einen Zielteil eines Patienten angewendet wird, zu berechnen, wobei die Vorrichtung Folgendes umfasst:
eine Speichereinheit (7), die ausgelegt ist, Daten bezüglich jeder der Dosisverteilungen, die erhalten werden, wenn ein Modell eines menschlichen Körpers für einen vorbestimmten Körpertyp mit einer Strahlung in Bezug auf mehrere Bestrahlungsbedingungen bestrahlt wird, zu speichern;
eine Eingabeeinheit (3), die ausgelegt ist, eine Bestrahlungsbedingung, die auf den Patienten angewendet wird, wenn der Patient mit Strahlung bestrahlt wird, und Körpertypinformationen (M₁) des Patienten einzugeben; und
eine Korrektureinheit (6), die ausgelegt ist, Daten bezüglich einer Dosisverteilung, die in der Speichereinheit (7) gespeichert sind, auf Grund der Körpertypinformationen (M₁) des Patienten und von Körpertypinformationen (M₀) des Modells des menschlichen Körpers (M₀) zu korrigieren,
wobei die Korrektureinheit (6) ausgelegt ist, Bildinformationen, die die Körpertypinformationen (M₁) des Patienten enthalten, mit den Körpertypinformationen (M₀) des Modells des menschlichen Körpers zu vergleichen, wobei die Bildinformationen durch Abbildungsvorrichtungen wie etwa CT aufgenommen werden, um Positionsinformationen in den Körpertypinformationen (M₀) des Modells des menschlichen Körpers in Positionsinformationen in den Körpertypinformationen (M₁) des Patienten umzuwandeln und die Daten bezüglich der Dosisverteilung, die in der Speichereinheit (7) gespeichert sind, gemäß der Umwandlung der Positionsinformationen zu korrigieren.

2. Strahlungstherapie-Simulationsvorrichtung (1) nach Anspruch 1,
wobei die Korrektureinheit (6) ausgelegt ist, Positionsinformationen bezüglich der Bestrahlungsbedingung, die auf den Patienten angewendet wird, in Positionsinformationen in den Körpertypinformationen (M₀) des Modells des menschlichen Körpers (M0) umzuwandeln, um Daten bezüglich einer Dosisverteilung, die den umgewandelten Positionsinformationen entspricht, aus den Daten bezüglich der Dosisverteilung, die in der Speichereinheit (7) gespeichert sind, zu extrahieren und die extrahierten Daten bezüglich der Dosisverteilung durch Umwandeln der Daten in Positionsinformationen auf Grund der Körpertypinformationen (M₁) des Patienten zu korrigieren.

## Revendications

1. Un appareil de simulation de radiothérapie (1) adapté pour calculer une distribution de dose sur la base d'une irradiation appliquée à une partie cible d'un patient, l'appareil comprenant :
une unité de stockage (7) adaptée pour stocker des données concernant chacune des distributions de doses obtenues lorsqu'un modèle de corps humain pour un type de corps prédéterminé est irradié avec un rayonnement par rapport à une pluralité de conditions d'irradiation ;
une unité d'entrée (3) adaptée pour entrer une condition d'irradiation appliquée au patient lorsque le patient est irradié avec le rayonnement, et des informations de type de corps (M₁) du patient ; et
une unité de correction (6) adaptée pour corriger des données concernant une distribution de dose stockées dans l'unité de stockage (7) sur la base des informations de type de corps (M₁) du patient et des informations de type de corps (M₀) du modèle de corps humain,
dans lequel l'unité de correction (6) est adaptée pour comparer des informations d'image incluant des informations de type de corps (M₁) du patient avec les informations de type de corps (M₀) du modèle de corps humain, les informations d'image étant capturées par des appareils d'imagerie tels que tomodensitomètre (CT), pour transformer des informations de position dans les informations de type de corps (M₀) du modèle de corps humain en informations de position dans les informations de type de corps (M₁) du patient, et pour corriger les données concernant la distribution de dose stockées dans l'unité de stockage (7) selon la transformée des informations de position.

2. Un appareil de simulation de radiothérapie (1) selon la revendication 1,
dans lequel l'unité de correction (6) est adaptée pour transformer des informations de position concernant la condition d'irradiation appliquée au patient en informations de position dans les informations de type de corps (M₀) du modèle de corps humain, pour extraire des données concernant une distribution de dose correspondant aux informations de position transformées à partir des données concernant la distribution de dose stockées dans l'unité de stockage (7), et pour corriger les données extraites concernant la distribution de dose en transformant les données en informations de position sur la base des informations de type de corps (M₁) du patient.
